# EUROPEAN PATENT APPLICATION

(11) **EP 2 787 079 A1**
(43) Date of publication of application: **08.10.2014**
(21) Application number: 12852729.8
(22) Date of filing: 29.11.2012
(51) Int. Cl.: C12N 15/09, C07K 1/22, C07K 7/08, C07K 16/00, C07K 17/00, C07K 19/00, G01N 33/53

(54) **IgA-BINDING PEPTIDE AND PURIFICATION OF IgA THEREBY**

(30) Priority: 30.11.2011 JP 2011262871
(71) Applicant: Kagoshima University, Kagoshima-shi, Kagoshima 890-8580 (JP); Otsuka Chemical Co., Ltd., Osaka-shi, Osaka 540-0021 (JP)
(72) Inventor: ITO, Yuji, Kagoshima 8908580 (JP); ITO, Osamu, Tokushima 7710193 (JP); OOZONO, Shinji, Tokushima 7710193 (JP); ISHITOBI, Hiroyuki, Tokushima 7710193 (JP)
(74) Representative: Campbell, Patrick John Henry
(86) International application number: PCT/JP2012/080875
(87) International publication number: WO 2013/081037

(57) **Abstract**

This invention provides a peptide that comprises an amino acid sequence consisting of 14 to 18 amino acid residues represented by Formula (I) and is capable of binding to human IgA:

H-(X₁)-V-C-L-S-Y-R-(X₂)-(X₃)-G-(X₄)-P-(X₅)-C-(X₆)-(X₇)-(X₈) (I) (SEQ ID NO: 1)

wherein each X is independently selected from among the following: X₁ represents a Gln residue or a Met residue; X₂ and X₃ each independently represent an arbitrary amino acid residue other than Cys or either or both thereof are deleted; X₄ represents an Arg residue or a Gln residue; X₅ represents a Val residue or a Thr residue; X₆ represents a Phe residue or a Tyr residue; X₇ represents a Ser residue or is null; and X₈ represents a Leu residue, a Thr residue, or is null, provided that, when X₁ represents a Met residue, X₇ represents a Ser residue or is null, and X₈ is null. The invention also provides a method for purifying or analyzing human IgA using such peptide.

SEQUENCE LISTING

<110>  Kagoshima University
       Otsuka Chemical Co., Ltd.

<120>  IgA-binding peptide and purification of IgA using same

<130>  PH-5454-PCT

<150>  JP 2011-262871
<151>  2011-11-30

<160>  13

<170>  PatentIn version 3.5

<210>  1
<211>  18
<212>  PRT
<213>  Artificial

<220>
<223>  IgA-binding peptide


<220>
<221>  misc_feature
<222>  (2)..(2)
<223>  Xaa is Gln or Met

<220>
<221>  misc_feature
<222>  (9)..(10)
<223>  Xaa is independently any amino acid residue other than Cys or one or
both are null

<220>
<221>  misc_feature
<222>  (12)..(12)
<223>  Xaa is Arg or Gln

<220>
<221>  misc_feature
<222>  (14)..(14)
<223>  Xaa is Val or Thr

<220>
<221>  misc_feature
<222>  (16)..(16)
<223>  Xaa is Phe or Tyr

<220>
<221>  misc_feature
<222>  (17)..(17)
<223>  Xaa is Ser or null (with the provision that when X1 is Met, X7 is Ser
or null and X8 is null)

<220>
<221>  misc_feature
<222>  (18)..(18)
<223>  Xaa is Leu, Thr or null (with the provision that when X1 is Met, X7
is Ser or null and X8 is null)

<400>  1

His Xaa Val Cys Leu Ser Tyr Arg Xaa Xaa Gly Xaa Pro Xaa Cys Xaa
1               5                   10                  15

Xaa Xaa

<210>  2
<211>  18
<212>  PRT
<213>  Artificial

<220>
<223>  IgA-binding peptide

<220>
<221>  misc_feature
<222>  (9)..(10)
<223>  Xaa is independently any amino acid residue other than Cys or one or
both are null

<220>
<221>  misc_feature
<222>  (12)..(12)
<223>  Xaa is Arg or Gln

<220>
<221>  misc_feature
<222>  (14)..(14)
<223>  Xaa is Val or Thr

<220>
<221>  misc_feature
<222>  (16)..(16)
<223>  Xaa is Phe or Tyr

<220>
<221>  misc_feature
<222>  (18)..(18)
<223>  Xaa is Leu or Thr

<400>  2

His Gln Val Cys Leu Ser Tyr Arg Xaa Xaa Gly Xaa Pro Xaa Cys Xaa
1               5                   10                  15

Ser Xaa

<210>  3
<211>  14
<212>  PRT
<213>  Artificial

<220>
<223>  IgA-binding peptide

<400>  3

His Met Val Cys Leu Ser Tyr Arg Gly Arg Pro Val Cys Phe
1               5                   10


<210>  4
<211>  15
<212>  PRT
<213>  Artificial

<220>
<223>  IgA-binding peptide

<400>  4

His Met Val Cys Leu Ser Tyr Arg Gly Arg Pro Val Cys Phe Ser
1               5                   10                  15


<210>  5
<211>  16
<212>  PRT
<213>  Artificial

<220>
<223>  IgA-binding peptide

<400>  5

His Gln Val Cys Leu Ser Tyr Arg Gly Arg Pro Val Cys Phe Ser Leu
1               5                   10                  15


<210>  6
<211>  16
<212>  PRT
<213>  Artificial

<220>
<223>  IgA-binding peptide

<400>  6

His Gln Val Cys Leu Ser Tyr Arg Gly Gln Pro Val Cys Phe Ser Leu
1               5                   10                  15


<210>  7
<211>  16
<212>  PRT
<213>  Artificial

<220>
<223>  IgA-binding peptide

<400>  7

His Gln Val Cys Leu Ser Tyr Arg Gly Arg Pro Thr Cys Phe Ser Leu
1               5                   10                  15


<210>  8

<211>  16
<212>  PRT
<213>  Artificial

<220>
<223>  IgA-binding peptide

<400>  8

His Gln Val Cys Leu Ser Tyr Arg Gly Arg Pro Val Cys Tyr Ser Leu
1               5                   10                  15


<210>  9
<211>  16
<212>  PRT
<213>  Artificial

<220>
<223>  IgA-binding peptide

<400>  9

His Gln Val Cys Leu Ser Tyr Arg Gly Arg Pro Val Cys Phe Ser Thr
1               5                   10                  15


<210>  10
<211>  16
<212>  PRT
<213>  Artificial

<220>
<223>  IgA-binding peptide

<400>  10

His Gln Val Cys Leu Ser Tyr Arg Gly Gln Pro Val Cys Phe Ser Thr
1               5                   10                  15


<210>  11
<211>  16
<212>  PRT
<213>  Artificial

<220>
<223>  IgA-binding peptide

<400>  11

His Gln Val Cys Leu Ser Tyr Arg Gly Arg Pro Thr Cys Phe Ser Thr
1               5                   10                  15


<210>  12
<211>  16
<212>  PRT
<213>  Artificial

<220>
<223>  IgA-binding peptide

<400>  12

His Gln Val Cys Leu Ser Tyr Arg Gly Gln Pro Thr Cys Phe Ser Thr
1               5                   10                  15

<210>  13
<211>  16
<212>  PRT
<213>  Artificial

<220>
<223>  IgA-binding peptide

<400>  13

His Met Val Cys Leu Ser Tyr Arg Gly Arg Pro Val Cys Phe Ser Leu
1               5                   10                  15

## Description

### Technical Field

The present invention relates to a human IgA-binding peptide obtained from a random peptide library and a method for analyzing or purifying IgA using such peptide.

### Background Art

Immunoglobulin A (IgA) is not only an important antibody for mucosal immunity; it also constitutes the second most major antibody class following immunoglobulin G (IgG) in blood, which defends against bacterial or viral infections. IgA includes secretory IgA (sIgA) having a dimeric structure and IgA having a monomeric structure (mIgA). sIgA has a dimeric structure in which the monomeric units are linked by a joining chain (J-chain) through disulfide bonds and it is secreted into mucus, while mIgA is mostly found in blood. Also, IgA has two subtypes: IgA1 and IgA2, which differ mainly in the length of the hinge region. IgA2 lacks a Pro-rich region of 13 residues. Attention regarding the functions of IgA directed to pharmaceuticals has been paid to the development of mucosal vaccines because of its importance for immunity against infections (Non-Patent Documents 1 and 2). IgA in blood has been reported to have ADCC against cancer cells particularly mediated by neutrophils (Non-Patent Documents 3 and 4). IgG is a form of an antibody drug, and its clinical applications are expanding as a therapeutic drug for cancer and autoimmune disease. As with the case of IgG, IgA can also be expected to be used as a cancer-targeting antibody drug (Non-Patent Document 5).

However, there are some impediments to the pharmaceutical development of IgA, including the absence of a purification means that can be employed at the industrial or pharmaceutical level, as with the case of protein A/G affinity columns for IgG production. Some methods have previously been reported as methods for purifying IgA (Non-Patent Document 6). The reported methods for purifying IgA utilize, for example, Jackalin, a lectin recognizing an IgA1-specific sugar chain (Non-Patent Document 7) or a protein A-mimetic synthetic ligand TG19318 (Non-Patent Document 8). Use of these methods is limited due to problems associated with binding ability and specificity. IgA-binding proteins have been found among members of the family of M proteins (Non-Patent Document 9), the surface proteins derived from *Streptococcus* bacteria (Non-Patent Documents 10 and 11 and Patent Document 1). These IgA-binding proteins, however, are problematic since they interact with other proteins in serum, such as IgG (Non-Patent Document 12). Accordingly, use thereof as IgA-specific affinity ligands has been difficult. Meanwhile, Sandin et al. reported that they isolated a domain peptide (*Streptococcal* IgA-binding peptide, Sap) consisting of 48 residues in the *Streptococcal* Sir22 (M22) protein and formed a disulfide-bonded dimer thereof via Cys to obtain an affinity ligand for IgA purification having relatively high affinity (Kd: 20 nM), although such affinity was lower than the affinity of the original Sir22 protein (Kd: 3 to 4 nM; Non-Patent Document 13). In fact, this ligand was capable of binding to IgA Fc and thus was applicable to purification of both sIgA and mIgA and to detection of antigen-specific IgA1 and IgA2 monoclonal antibodies.

As in the case of IgA-binding proteins, the present inventors have also developed IgG-binding peptides (Patent Document 2). However, IgG-binding peptides bind specifically to IgG, and they cannot be used as IgA-binding peptides. In order to develop peptides that bind specifically to IgA, it was necessary to adopt an approach that is completely different from the approach used to develop IgG-binding peptides because of differences in specificity.

Accordingly, a novel technique for purification or analysis (detection or quantification) of IgA has been awaited in the art.

### Prior Art Documents

### Patent Documents:

Patent Document 1: International Publication No. WO 1992/017588
Patent Document 2: International Publication No. WO 2000/063383

### Non-Patent Documents:

Non-Patent Document 1: Holmgren, J., 1991, Fems Microbiology Immunology 89 (1), 1-9
Non-Patent Document 2: Holmgren, J. and Czerkinsky, C., 2005, Nat. Med. 11 (4), S45-S53
Non-Patent Document 3: Dechant, M., Beyer, T., Schneider-Merck, T., Weisner, W, Peipp, M., van de Winkel, J. G., and Valerius, T., 2007, J. Immunol. 179 (5), 2936-2943
Non-Patent Document 4: Zhao, J., Kuroki, M., Shibaguchi, H., Wang, L., Huo, Q., Takami, N., Tanaka, T., Kinugasa, T., and Kuroki, M., 2008, Oncol. Res. 17 (5), 217-222
Non-Patent Document 5: Beyer, T., Lohse, S., Berger, S., Peipp, M., Valerius, T., and Dechant, M., 2009, Journal of Immunological Methods 346 (1-2), 26-37
Non-Patent Document 6: Pack, T. D., 2001, Current protocols in Immunology / edited by John E. Coligan et al., Chapter 2, Unit 2 10B
Non-Patent Document 7: Kondoh, H., Kobayashi, K., and Hagiwara, K., 1987, Molecular immunology 24 (11), 1219-1222
Non-Patent Document 8: Palombo, G., De Falco, S., Tortora, M., Cassani, G., and Fassina, G., 1998, J. Mol. Recognit. 11 (1-6), 243-246
Non-Patent Document 9: Frithz, E., Heden, L. O., and Lindahl, G., 1989, Molecular Microbiology 3 (8), 1111-1119
Non-Patent Document 10: Russell-Jones, G. J., Gotschlich, E. C., and Blake, M. S., 1984, Journal of Experimental Medicine 160 (5), 1467-1475
Non-Patent Document 11: Lindahl, G., Akerstrom, B., Vaerman, J. P., and Stenberg, L., 1990, European Journal of Immunology 20 (10), 2241-2247
Non-Patent Document 12: Stenberg, L., O'Toole, P. W., Mestecky, J., and Lindahl, G., 1994, Journal of Biological Chemistry 269 (18), 13458-13464
Non-Patent Document 13: Sandin, C., Linse, S., Areschoug, T., Woof, J. M., Reinholdt, J., and Lindahl, G., 2002, J. Immunol. 169 (3), 1357-1364

### Summary of the Invention

### Objects to Be Attained by the Invention

An object of the present invention is to provide a peptide capable of specifically or selectively binding to human IgA.

Another object of the present invention is to provide a method for purifying or analyzing (e.g., detecting or quantifying) human IgA using such peptide.

### Means for Attaining the Objects

As described in the "Background Art" section above, human IgA is present in mucosa and blood, and it plays a key role in defense against infections, etc. Because of such properties, IgA is to be used as an antibody drug in the treatment of disease, such as infectious diseases or tumors. In consideration of such circumstances, the present invention provides a peptide capable of specifically or selectively binding to human IgA, and such peptide can be useful for purification and analysis of IgA that can be used for pharmaceutical applications.

In short, the present invention has the following features.
[1] A peptide, which comprises an amino acid sequence consisting of 14 to 18 amino acid residues represented by Formula (I) and is capable of binding to human IgA:

   H-(X₁)-V-C-L-S-Y-R-(X₂)-(X₃)-G-(X₄)-P-(X₅)-C-(X₆)-(X₇)-(X₈) (I) (SEQ ID NO: 1)

   wherein
   H represents a histidine residue,
   V represents a valine residue,
   C represents a cysteine residue,
   L represents a leucine residue,
   S represents a serine residue,
   Y represents a tyrosine residue,
   R represents an arginine residue,
   G represents a glycine residue,
   P represents a proline residue, and
   each X is independently selected from among the following:
      X₁ represents a glutamine residue or a methionine residue;
      X₂ and X₃ each independently represent an arbitrary amino acid residue other than cysteine or either or both thereof are deleted;
      X₄ represents an arginine residue or a glutamine residue;
      X₅ represents a valine residue or a threonine residue;
      X₆ represents a phenylalanine residue or a tyrosine residue;
      X₇ represents a serine residue or is null; or
      X₈ represents a leucine residue, a threonine residue, or is null,
   provided that, when X₁ represents a methionine residue, X₇ represents a serine residue or is null, and X₈ is null.
[2] The peptide according to [1], which comprises an amino acid sequence consisting of 16 to 18 amino acid residues represented by Formula (II) and is capable of binding to human IgA:

   H-Q-V-C-L-S-Y-R-(X₂)-(X₃)-G-(X₄)-P-(X₅)-C-(X₆)-S-(X₈) (II) (SEQ ID NO: 2)

   wherein
   H represents a histidine residue,
   Q represents a glutamine residue,
   V represents a valine residue,
   C represents a cysteine residue,
   L represents a leucine residue,
   S represents a serine residue,
   Y represents a tyrosine residue,
   R represents an arginine residue,
   G represents a glycine residue,
   P represents a proline residue,
   F represents a phenylalanine residue, and
   each X is independently selected from among the following:
      X₂ and X₃ each independently represent an arbitrary amino acid residue other than cysteine or either or both thereof are deleted;
      X₄ represents an arginine residue or a glutamine residue,
      X₅ represents a valine residue or a threonine residue,
      X₆ represents a phenylalanine residue or a tyrosine residue, or
      X₈ represents a leucine residue or a threonine residue.
[3] The peptide according to [1] consisting of either of the following amino acid sequences:
   HMVCLSYRGRPVCF (SEQ ID NO: 3); or
   HMVCLSYRGRPVCFS (SEQ ID NO: 4).
[4] The peptide according to [1] or [2] consisting of any of the amino acid sequences 1) to 8):
   1) HQVCLSYRGRPVCFSL (SEQ ID NO: 5);
   2) HQVCLSYRGQPVCFSL (SEQ ID NO: 6);
   3) HQVCLSYRGRPTCFSL (SEQ ID NO: 7);
   4) HQVCLSYRGRPVCYSL (SEQ ID NO: 8);
   5) HQVCLSYRGRPVCFST (SEQ ID NO: 9);
   6) HQVCLSYRGQPVCFST (SEQ ID NO: 10);
   7) HQVCLSYRGRPTCFST (SEQ ID NO: 11); or
   8) HQVCLSYRGQPTCFST (SEQ ID NO: 12).
[5] The peptide according to any of [1] to [4], which forms a disulfide bond between two cysteine (C) residues.
[6] The peptide according to any of [1] to [5], which binds to serum (monomeric) IgA and secretory (dimeric) IgA.
[7] The peptide according to any of [1] to [6] comprising a label bound thereto.
[8] A fusion protein comprising the peptide according to any of [1] to [7] and a protein which is linked to the peptide.
[9] An immobilized peptide, which comprises the peptide according to any of [1] to [7] bound to a solid phase.
[10] A nucleic acid encoding the peptide according to any of [1] to [7].
[11] A method for purifying IgA comprising binding the peptide according to any of [1] to
[7] or the immobilized peptide according to [9] to IgA and releasing the bound IgA to collect the released IgA.
[12] A method for detecting IgA comprising binding IgA in a sample to the peptide according to any of [1] to [7] or the immobilized peptide according to [9] and detecting the bound IgA.
[13] A kit for analyzing or purifying human IgA comprising at least one of the peptides according to any of [1] to [7] or the immobilized peptide according to [9].
[14] An IgA separation column comprising the immobilized peptide according to [9].

This description contains part or all of the content as disclosed in the description and/or drawings of Japanese Patent Application No. 2011-262871, based on which the present application claims priority.

### Effects of the Invention

The human IgA-binding peptide of the present invention is capable of binding to human IgA with higher selectivity than to IgG, IgM, or IgE. This indicates that use of such peptide enables selective separation of IgA from human serum, for example.

### Brief Description of Drawings

Fig. 1 shows the results of surface plasmon analysis of binding of the synthetic peptide (i.e., Opt2 M2Q R10Q; SEQ ID NO: 6) to human IgA.
Fig. 2 shows the results of surface plasmon analysis of binding of the synthetic peptide (i.e., Opt2 M2Q V12T; SEQ ID NO: 7) to human IgA.
Fig. 3 shows the results of surface plasmon analysis of binding of the synthetic peptide (i.e., Opt2 M2Q L16T; SEQ ID NO: 9) to human IgA.
Fig. 4 shows purification of IgA from human serum using a column on which the Opt2 M2Q R10Q peptide (SEQ ID NO: 6) is immobilized (the amount of peptide immobilized: 458.62 nmol).
Fig. 5 shows purification of IgA from human serum using a column on which the Opt2 M2Q V12T peptide (SEQ ID NO: 7) is immobilized (the amount of peptide immobilized: 490.06 nmol).
Fig. 6 shows purification of IgA from human serum using a column on which the Opt2 M2Q L16T peptide (SEQ ID NO: 9) is immobilized (the amount of peptide immobilized: 515.32 nmol).
Fig. 7 shows the results of SDS-PAGE (A) and Western blotting (B) of IgA collected from the column on which the peptide is immobilized.

### Embodiments for Carrying out the Invention

The peptides capable of specifically or selectively binding to human IgA that were discovered by the present inventors were isolated by a biopanning method from a new library designed and constructed with reference to a random peptide library (Sakamoto, K., Ito, Y, Hatanaka, T., Soni, P. B., Mori, T., and Sugimura, K., 2009, the Journal of Biological Chemistry 284 (15), 9986-9993) including peptides each containing one intramolecular disulfide bond constructed with the T7 phage display system. Four specific clones obtained by this method exhibited sequence homology common to each other. Synthetic peptides prepared by various substitutions or deletions on the basis of their sequences exhibited specificity for IgA. Residues of these peptides essential for binding to IgA were identified to achieve an approach to enhance affinity and application for purification of IgA from human serum using such peptides. The smallest IgA-binding peptide of the present invention consists of 14 residues and is smaller than the *Streptococcus* Sir22 (M22)-derived Sap peptide of approximately 50 residues (having one Cys residue at the C-terminal end) described in Non-Patent Document 13. As a result, construction of an IgA purification system at low cost based on these peptides can be expected.

Hereinafter, the present invention is described in greater detail.

Specifically, the IgA-binding peptide of the present invention, a method for purifying or analyzing IgA using such peptide, and a kit for purifying or detecting IgA are described.

### (IgA-binding peptide)

The peptides of the present invention were obtained by screening a phage library containing a large number of random peptides for specific or selective binding to human IgA. The peptides of the present invention differ in their origins and primary structures from conventional polypeptides known in the art as described in Non-Patent Document 13.

The human IgA used in the present specification refers to IgA1 or IgA2.

Specifically, the peptide of the present invention comprises an amino acid sequence consisting of 14 to 18 amino acid residues represented by Formula (I) in terms of the primary structure in the broadest sense and is capable of binding to human IgA:

H-(X₁)-V-C-L-S-Y-R-(X₂)-(X₃)-G-(X₄)-P-(X₅)-C-(X₆)-(X₇)-(X₈) (I) (SEQ ID NO: 1)

wherein
H represents a histidine residue,
V represents a valine residue,
C represents a cysteine residue,
L represents a leucine residue,
S represents a serine residue,
Y represents a tyrosine residue,
R represents an arginine residue,
G represents a glycine residue,
P represents a proline residue, and
each X is independently selected from among the following:
   X₁ represents a glutamine residue or a methionine residue;
   X₂ and X₃ each independently represent an arbitrary amino acid residue other than cysteine or either or both thereof are deleted;
   X₄ represents an arginine residue or a glutamine residue,
   X₅ represents a valine residue or a threonine residue,
   X₆ represents a phenylalanine residue or a tyrosine residue,
   X₇ represents a serine residue or is null, or
   X₈ represents a leucine residue, a threonine residue, or is null,
provided that, when X₁ represents a methionine residue, X₇ represents a serine residue or is null, and X₈ is null.

Two cysteine residues in Formula (I) can be disulfide-bonded to form a cyclic peptide. Typically, the peptide of Formula (I) has this disulfide bond.

A peptide represented by Formula (II) comprising an amino acid sequence derived from the amino acid sequence represented by Formula (I) in which amino acid residue Xs are further defined is shown below.

Specifically, a peptide represented by Formula (II) comprises an amino acid sequence consisting of 16 to 18 amino acid residues and is capable of binding to human IgA:

H-Q-V-C-L-S-Y R-(X₂)-(X₃)-G-(X₄)-P-(X₅)-C-(X₆)-S-(X₈) (II) (SEQ ID NO: 2)

wherein
H represents a histidine residue,
Q represents a glutamine residue,
V represents a valine residue,
C represents a cysteine residue,
L represents a leucine residue,
S represents a serine residue,
Y represents a tyrosine residue,
R represents an arginine residue,
G represents a glycine residue,
P represents a proline residue,
F represents a phenylalanine residue, and
each X is independently selected from among the following:
   X₂ and X₃ each independently represent an arbitrary amino acid residue other than cysteine or either or both thereof are deleted;
   X₄ represents an arginine residue or a glutamine residue;
   X₅ represents a valine residue or a threonine residue;
   X₆ represents a phenylalanine residue or a tyrosine residue; or
   X₈ represents a leucine residue or a threonine residue.

Preferably, both amino acid residue Xs at the 9th and 10th positions from the N terminus are deleted when the number of amino acid residues in each of the amino acid sequences of the peptides represented by Formulae (II) to (IV) is 18. Such a peptide consists of 16 amino acid residues.

Specific examples of the peptides of the present invention 1) to 10) are shown below, although the peptides of the present invention are not limited thereto. All such peptides have much higher binding specificity or binding selectivity for human IgA than that for immunoglobulins of other species.
1) HMVCLSYRGRPVCF (SEQ ID NO: 3)
2) HMVCLSYRGRPVCFS (SEQ ID NO: 4)
3) HQVCLSYRGRPVCFSL (SEQ ID NO: 5)
4) HQVCLSYRGQPVCFSL (SEQ ID NO: 6)
5) HQVCLSYRGRPTCFSL (SEQ ID NO: 7)
6) HQVCLSYRGRPVCYSL (SEQ ID NO: 8)
7) HQVCLSYRGRPVCFST (SEQ ID NO: 9)
8) HQVCLSYRGQPVCFST (SEQ ID NO: 10)
9) HQVCLSYRGRPTCFST (SEQ ID NO: 11)
10) HQVCLSYRGQPTCFST (SEQ ID NO: 12)

The peptides of SEQ ID NO: 6 (Opt2 M2Q R10Q), SEQ ID NO: 7 (Opt2 M2Q V12T), and SEQ ID NO: 9 (Opt2 M2Q L16T) have particularly high affinity for human IgA, as described in the examples below in greater detail.

As described above, each of the peptides represented by the formulae according to the present invention has two discrete cysteine (C) residues in its amino acid sequence. The peptides are characterized in that these cysteine residues are arranged such that a disulfide bond forms between such cysteine residues. A preferable peptide is a cyclic peptide formed through a disulfide bond between two cysteine residues, and such peptide comprises, on the N-terminal or C-terminal side of each cysteine residue, 1 to 3, and preferably 3, arbitrary amino acid residues other than cysteine. The 1st to the 3rd and the 16th to the 18th amino acid residues are as exemplified above.

The peptides of the present invention have binding affinity for human IgA that is about 10 times, preferably about 50 times, and more preferably about 200 times or more that for other human immunoglobulins (IgG, IgE, and IgM). The dissociation constant (Kd) in the binding of the peptides of the present invention with human IgA can be determined by surface plasmon resonance spectrum analysis (using, e.g., the BIACORE system). For example, it is less than 1x10⁻⁶ M to less than 1x10⁻⁷ M, and preferably less than 1x10⁻⁸ M.

The peptide of the present invention immobilized on a solid phase was actually used in an attempt of binding to IgA in human serum, and, as a consequence, the peptide was found to bind to serum (monomeric) IgA and secretory (dimeric) IgA. This demonstrates that both forms of IgA can be separated.

The peptide of the present invention can be produced by, for example, a common peptide synthesis method, such as a liquid-phase or solid-phase synthesis method, or peptide synthesis using an automatic peptide synthesizer (Kelley et al., Genetics Engineering Principles and Methods, Setlow, J. K. eds., Plenum Press NY, 1990, Vol. 12, pp. 1-19; Stewart et al., Solid-Phase Peptide Synthesis, 1989, W. H. Freeman Co.; Houghten, Proc. Natl. Acad. Sci., U.S.A., 1985, 82: p. 5132; and *"*Shin Seikagaku Jikken Koza 1 (New Biochemistry Experimental Lecture 1), Protein IV," 1992, edited by the Japanese Biochemical Society, Tokyo Kagaku Dojin Co., Ltd.). Alternatively, the peptide of the present invention may be produced by a genetic recombination method or a phage display method using a nucleic acid encoding the peptide. For example, DNA encoding the amino acid sequence of the peptide of the present invention is incorporated into an expression vector, and the resultant is introduced into a host cell, followed by culture. Thus, the peptide of interest can be produced. The produced peptide can be collected or purified by a conventional technique, such as chromatography (e.g., gel filtration chromatography, ion-exchange column chromatography, affinity chromatography, reverse-phase column chromatography, or HPLC), ammonium sulfate fractionation, ultrafiltration, or immunoadsorption.

For peptide synthesis, amino acids in which functional groups other than α-amino and α-carboxyl groups to be bound in each amino acid have been protected are prepared. The α-amino group of one amino acid is allowed to form a peptide bond with the α-carboxyl group of another amino acid. Typically, the carboxyl group of the amino acid residue to be positioned at the C terminus of the peptide is bound, in advance, to a solid phase via an appropriate spacer or linker. The protective group at the amino terminus of the dipeptide thus obtained is selectively removed, and the amino terminus forms a peptide bond with the α-carboxyl group of a subsequent amino acid. This operation is continuously performed to produce a peptide having protected side groups. Finally, all the protective groups are removed, and the resulting peptide is separated from the solid phase. The details of the types of the protective groups, protection methods, and peptide binding methods are specifically described in the documents mentioned above.

The genetic recombination method comprises inserting DNA encoding the peptide of the present invention into an appropriate expression vector, introducing the vector into an appropriate host cell, conducting cell culture, and collecting the peptide of interest from the cell or from extracellular fluid. Examples of the vectors include, but are not limited to, plasmid, phage, cosmid, phagemid, and viral vectors. Examples of the plasmid vectors include, but are not limited to, *E. coli*-derived plasmids (e.g., pET22b (+), pBR322, pBR325, pUC118, pUC119, pUC18, pUC19, and pBluescript), *Bacillus subtilis*-derived plasmids (e.g., pUB110 and pTP5), and yeast-derived plasmids (e.g., YEp13 and YCp50). Examples of the phage vectors include, but are not limited to, T7 phage display vectors (T7Select 10-3b, T7Select 1-1b, T7Select 1-2a, T7Select 1-2b, T7Select 1-2c, etc. (Novagen)) and λ phage vectors (Charon 4A, Charon 21A, EMBL3, EMBL4, λgt10, λgt11, λZAP, λZAP II, etc.). Examples of the viral vectors include, but are not limited to, animal viruses such as retrovirus, adenovirus, adeno-associated virus, vaccinia virus, and hemagglutinating virus of Japan, and insect viruses such as baculovirus. Examples of the cosmid vectors include, but are not limited to, Lorist 6, Charomid 9-20, and Charomid 9-42. Examples of the phagemid vectors include, but are not limited to, pSKAN, pBluescript, pBK, and pComb3H. Each vector may contain, for example, regulatory sequences that allow the expression of the DNA of interest, a selection marker for screening for a vector containing the DNA of interest, and a multicloning site into which the DNA of interest is inserted. Such regulatory sequences encompass promoters, enhancers, terminators, S-D sequences or ribosomal binding sites, replication origins, poly-A sites, and the like. For example, an ampicillin resistance gene, a neomycin resistance gene, a kanamycin resistance gene, or a dihydrofolate reductase gene may be used as a selection marker. The host cells into which the vectors are introduced are, for example, bacteria such as *E. coli* or *Bacillus subtilis,* yeast cells, insect cells, animal cells (e.g., mammalian cells), or plant cells. These cells are transformed or transfected by a method such as a calcium phosphate, electroporation, lipofection, particle gun, or PEG method. The transformed cells are cultured according to a method commonly used for culture of host organisms. For example, microbes such as *E. coli* or yeast cells are cultured in a medium containing a carbon source, a nitrogen source, inorganic salts, etc., assimilable by the host microbes. For easy collection of the peptide of the present invention, extracellular secretion of the peptide produced by expression is preferred. For this purpose, DNA encoding a peptide sequence that allows peptide secretion from the cells is bound to the 5' end of the DNA encoding the peptide of interest. A fusion peptide transferred to the cell membrane is cleaved by signal peptidase to secrete and release the peptide of interest into the medium. Alternatively, the intracellularly accumulated peptide of interest may be collected. In this case, the cells are physically or chemically disrupted, and the peptide of interest is collected therefrom using a protein purification technique.

Thus, the present invention further relates to a nucleic acid encoding the peptide of the present invention. In this context, the nucleic acid encompasses DNA and RNA (e.g., mRNA).

The peptide of the present invention may be labeled in order to enable detection of IgA. Examples of labels include, but are not limited to, fluorescent dyes, chemiluminescent dyes, enzymes, radioisotopes, fluorescent proteins, and biotin. Preferable examples of labels include fluorescein, fluorescein derivatives such as FITC, rhodamine, rhodamine derivatives such as tetramethylrhodamine, and fluorescent dyes such as Texas Red.

The peptide of the present invention may be fused with an arbitrary protein. For example, a fluorescent protein such as GFP (green fluorescent protein) or an enzyme such as peroxidase may be used as a label. In this case, the peptide of the present invention and such protein can be prepared as a fusion protein via an appropriate linker by a genetic recombination method, according to need. In such a case, the fusion protein should be prepared without impairing the ability of the peptide of the present invention to bind to human IgA.

Also, the peptide of the present invention may be immobilized on a solid phase that can be filled in an affinity column, so that it can be used for separation, purification, analysis, or another form of processing of human IgA.

Examples of a solid phase that can be preferably used for peptide immobilization include, but are not limited to, polystyrene, polyethylene, polypropylene, polyester, polyacrylonitrile, styrene-butadiene copolymers, (meth)acrylic acid ester polymers, fluoropolymers, silica gels, saccharides such as cross-linked dextran, polysaccharide, and agarose, glass, metals, magnetic materials, and combinations of any thereof. Such solid phase may be in any form, such as a tray, sphere, fiber, particle, rod, flat plate, container, cell, microplate, test tube, film or membrane, gel, or chip. Specific examples thereof include magnetic beads, glass beads, polystyrene beads, Sepharose beads, silica gel beads, polysaccharide beads, polystyrene plates, glass plates, and polystyrene tubes. The peptide of the present invention can be immobilized on the solid phase by a method well known to a person skilled in the art. For example, physical adsorption, covalent binding, or ionic binding may be performed. Immobilization is preferably performed via covalent binding. The solid phase has, on its surface, a chemical functional group(s) (e.g., hydroxy, amino, and N-hydroxysuccinimidyl groups), and preferably a chemical functional group(s) comprising an alkylene chain having approximately 4 to 20 carbon atoms as a spacer, and the functional group(s) is allowed to chemically react with the carboxy terminus of the peptide, so as to form an ester bond, an amide bond, or the like. The solid phase onto which the peptide of the present invention is immobilized can be used to fill a column such as an affinity chromatography column or an HPLC column and then used for detection, purification, or separation of human IgA.

### (Method for purifying IgA)

The present invention further provides a method for purifying IgA comprising binding the peptide or immobilized peptide of the present invention to IgA and releasing the bound IgA to collect IgA.

The solid phase on which the peptide of the present invention is immobilized is used to fill a column such as an affinity chromatography column or an HPLC column. The column is equilibrated with an appropriate buffer. A solution containing human IgA is applied thereto at 40°C to 0°C, and preferably at room temperature, so as to bind human IgA to the peptide on the solid phase. When separating IgA from serum, for example, the serum can be applied to the column using a buffer having a neutral pH (e.g., a pH of 6.0 to 7.5), to bind human IgA to the peptide. A buffer having a pH in the acidic range, such as pH 2 to 4 (e.g., a 0.2 M glycine-HCl buffer (pH 3.5 to 2.5) containing 0.3 M NaCl) can be flushed through the column to elute IgA.

Whether or not IgA is collected can be determined by, for example, electrophoresis and subsequent Western blotting using anti-human IgA antibodies. Electrophoresis can be carried out via SDS-PAGE using a 5% to 20% acrylamide gradient gel. Western blotting can be carried out by transferring the proteins thus electrophoresed to a PVDF membrane, and blocking the membrane with skim milk, followed by detection using anti-human IgA α-chain goat antibodies and HRP-labeled anti-goat IgG mouse antibodies.

The method of the present invention is useful for obtaining an IgA-rich fraction in the step of purifying IgA from IgA-containing products formed by various methods. Accordingly, the method of the present invention is preferably employed for column chromatography such as affinity chromatography or HPLC. For IgA purification, such a chromatography method as well as protein purification techniques routinely used, such as chromatography (e.g., gel filtration chromatography, ion-exchange column chromatography, or reverse-phase column chromatography), ammonium sulfate fractionation, ultrafiltration, and the like, can be combined appropriately.

### (Method for analyzing IgA)

The present invention further provides a method for detecting IgA comprising binding the peptide or immobilized peptide of the present invention to IgA in a sample and detecting the bound IgA. In this method, detection involves qualitative or quantitative analysis.

IgA can be detected, with the use of a suitable buffer, by binding the sample to a membrane, polystyrene well plate, or the like, bringing the labeled peptide of the present invention into contact therewith, and washing the resultant according to need, followed by qualitative or quantitative analysis of the level of the label.

Alternatively, the HPLC column on which the peptide of the present invention is immobilized as described above may be used. In such a case, a sample containing human IgA is injected into the column, and the human IgA is allowed to bind to the peptide by flushing a binding buffer therethrough. The bound protein is detected and recorded with, for example, the absorbance at 280 nm or the fluorescence at 350 nm emitted by excitation at 280 nm and eluted from the column using an elution buffer (e.g., gradient elution in a 0.1 M glycine-HCl buffer containing 0.15 M NaCl, at pH 2.5). IgA can be analyzed qualitatively or quantitatively based on the peak observed and the peak area.

### (Kit and column)

The present invention further provides a kit for analysis (e.g., qualitative or quantitative analysis) or purification of human IgA comprising at least one of the peptide or immobilized peptide of the present invention.

Individual peptides or immobilized peptides contained in the kit of the present invention are accommodated in separate containers. If necessary, the kit may also contain instructions describing the procedures for analyzing or purifying human IgA. The kit may further contain reagents or buffers necessary for analysis, an immobilized peptide-packed column, etc.

The present invention further provides a column for IgA separation comprising the immobilized peptide of the present invention.

In general, the immobilized peptide may be prepared by binding the peptide covalently or non-covalently to a carrier (or a filler) for chromatography. Examples of carriers include polysaccharide-based carriers, such as agarose- or Sepharose-based carriers, silica gel-based carriers, and resin- or polymer-based carriers. The peptide may be bound to the carrier via a spacer such as a hydrocarbon chain (e.g., C4 to C16).

The IgA separation column is used for separating IgA. Specific examples thereof include chromatography columns and high-performance liquid chromatography (HPLC) columns used for analysis, purification, or fractionation of IgA. The size of the column is not particularly limited, and it may vary depending on its application (e.g., for analysis or for purification or fractionation), the amount thereof applied (loaded) or injected, and other conditions. The column may be made of a material usually used for a column, such as a metal, plastic, or glass.

The column can be produced by densely packing the immobilized peptide of the present invention (in a dry or wet state) prepared according to the technique described above into the column.

### Examples

Hereafter, the present invention is described in greater detail with reference to the examples, although the technical scope of the present invention is not limited to these examples.

### [Example 1]

### Evaluation of specificity of synthetic peptide

The peptides described below were synthesized in accordance with a known general technique. The sequences of these peptides were discovered by the method of the present inventors disclosed in PCT/JP2011/061906. Such sequences are derived from, in particular, the sequence of the IgA-binding peptide (i.e., Opt2: HMVCLSYRGRPVCFSL (SEQ ID NO: 13); SEQ ID NO: 44 in PCT/JP2011/061906) by substitution or deletion of particular amino acids for the purpose of improvement of hydrophilic properties or solubility of such peptides and attenuation of positive charges of such peptides.

**[Table 1]**

| Peptide | Sequence | | | | | | | | | | | | | | | | SEQ ID NO: | KD (µM) |
|---|---|---|---|---|---|---|---|---|---|---|---|---|---|---|---|---|---|---|
| | 1 | | | | 5 | | | | | 10 | | | | | 15 | | | |
| Opt2 14AA | H | M | V | C | L | S | Y | R | G | R | P | V | C | F | - | - | 3 | 0.603 |
| Opt2 15AA | H | M | V | C | L | S | Y | R | G | R | P | V | C | F | S | - | 4 | 0.491 |
| Opt2 M2Q | H | Q | V | C | L | S | Y | R | G | R | P | V | C | F | S | L | 5 | 0.023 |
| Opt2 M2Q R10Q | H | Q | V | C | L | S | Y | R | G | Q | P | V | C | F | S | L | 6 | 0.065 |
| Opt2 M2Q V12T | H | Q | V | C | L | S | Y | R | G | R | P | T | C | F | S | L | 7 | 0.149 |
| Opt2 M2Q F14Y | H | Q | V | C | L | S | Y | R | G | R | P | V | C | Y | S | L | 8 | 0.624 |
| Opt2 M2Q L16T | H | Q | V | C | L | S | Y | R | G | R | P | V | C | F | S | T | 9 | 0.072 |
| Opt2 M2Q L16T R10Q | H | Q | V | C | L | S | Y | R | G | Q | P | V | C | F | S | T | 10 | 0.699 |
| Opt2 M2Q L16T V12T | H | Q | V | C | L | S | Y | R | G | R | P | T | C | F | S | T | 11 | 0.952 |
| Opt2 M2Q L16T R10 Q V12T | H | Q | V | C | L | S | Y | R | G | Q | P | T | C | F | S | T | 12 | 2.82 |

In order to verify binding specificity of such peptides, binding strength was evaluated via surface plasmon resonance analysis using a BiaCore T100 (GE Healthcare).

Table 1 shows the binding affinity (KD) of each peptide to human IgA.

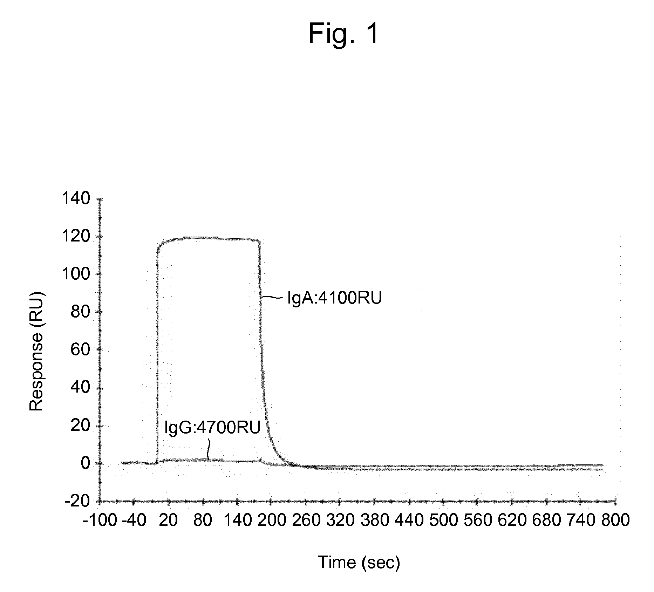

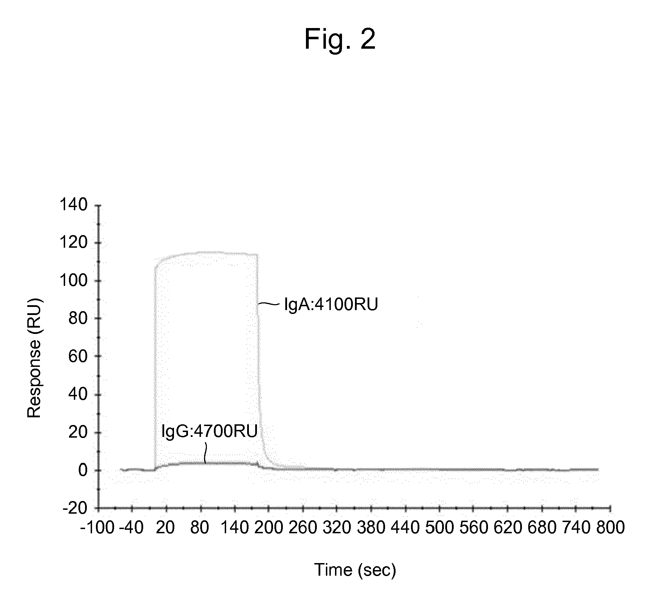

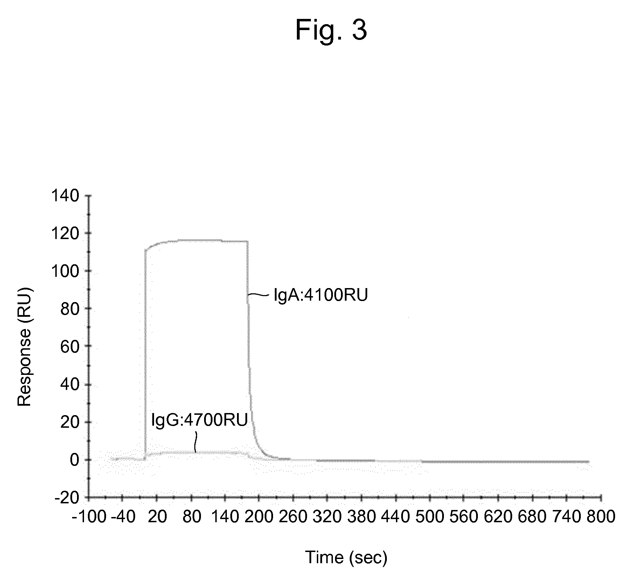

Figs. 1 to 3 show the results of analysis of binding specificity of representative peptides. The results demonstrate that none of the above peptides have affinity to IgG but specifically bind to IgA.

### [Example 2]

### Purification of human IgA by IgA-binding peptide

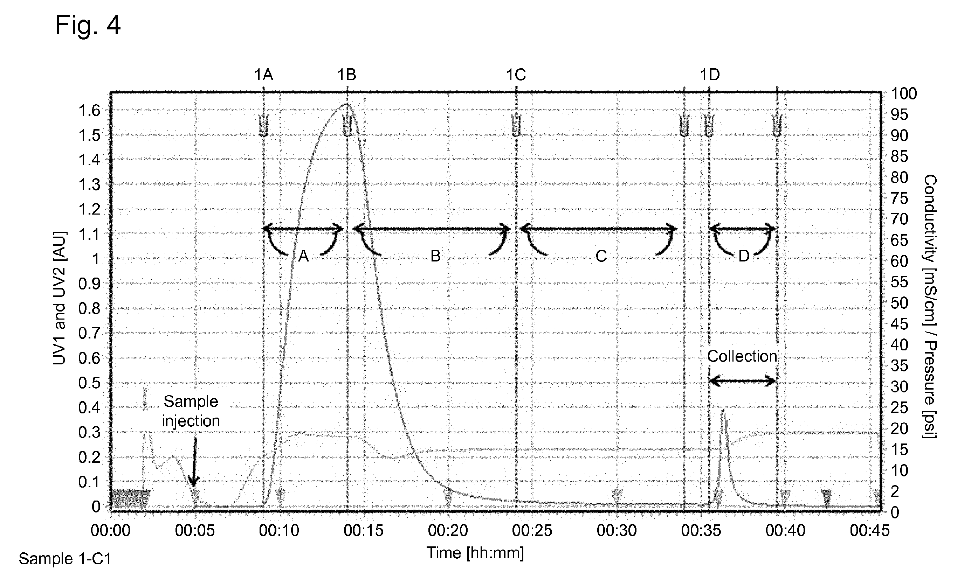

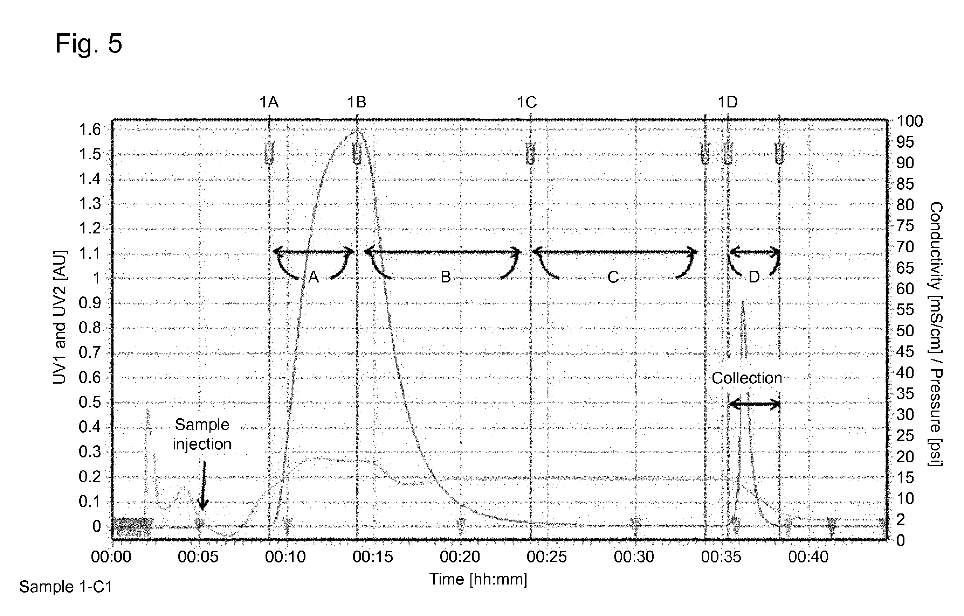

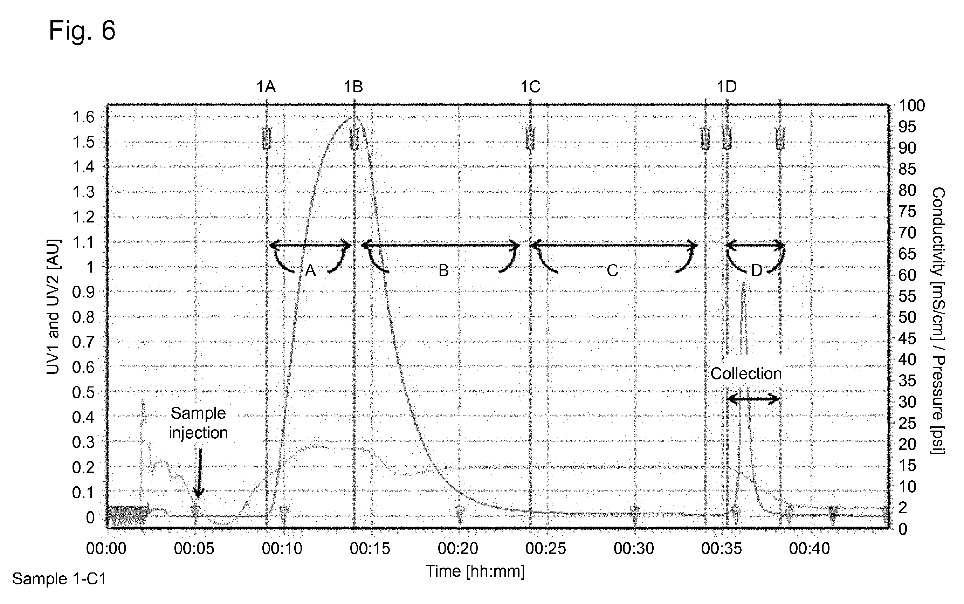

In order to examine whether or not the peptides synthesized in Example 1 would function as human IgA purification ligands, the synthesized peptides were subjected to amino-PEGylation, the resultants were immobilized to 1 ml of HiTrap NHS-activated HP (GE Healthcare), and purification of IgA from human serum was attempted. After 1 ml of human serum had been diluted 5-fold with PBS, the resultant was applied to a column connected to a protein purification system (Profinia, BioRad). The column was subjected to washing with PBS and the bound protein was then eluted using a 0.1 M glycine-HCl buffer (pH 2.5) containing 0.15 M NaCl. Figs. 4 to 6 show the results of elution of representative peptides. Similar results were obtained with the use of each of the peptides synthesized in Example 1, and fractions D were collected from among the fractions A to D.

### [Example 3]

### Identification of purified protein

Purified proteins were identified via SDS-PAGE.

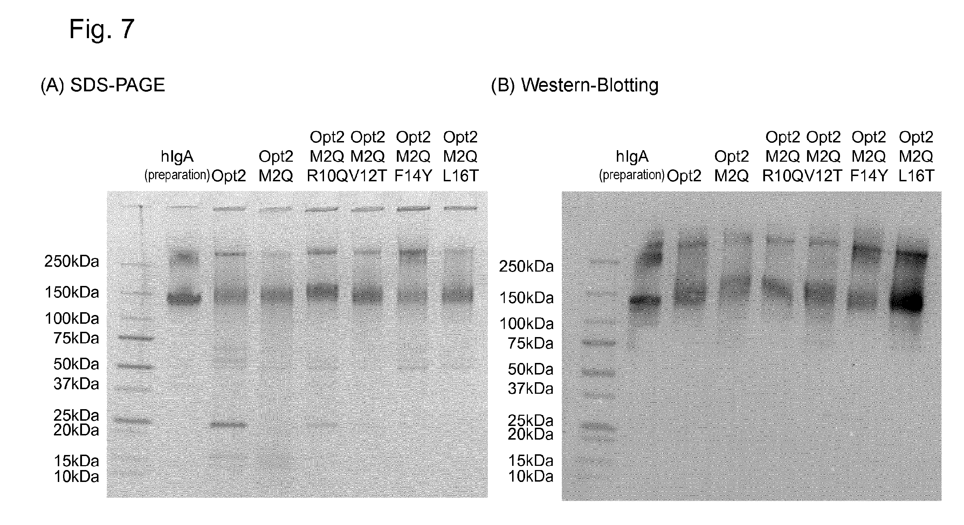

The fraction Ds obtained in Example 2 were diluted with a 137 mM PBS solution to adjust the concentration of each thereof to substantially the same level, and the resultants were further diluted 2-fold with the use of a 2x sample buffer (10 ml of a solution comprising 1.25 ml of 0.5 M Tris-HCl buffer (pH 6.8), 2 ml of glycerol, 0.2 g of SDS, and 1 ml of 0.1% BPB, with the balance consisting of ultrapure water) (mixing ratio: 1:1). The resulting samples (30 µl) were applied to 4% to 20% polyacrylamide gradient gel (Mini-PROTEAN TGX gels, BioRad), and the resultants were electrophoresed in a tank filled with electrode buffer (a solution of 9 g of Tris, 3 g of SDS, and 43.2 g of glycine in 3 liters of distilled water) at 35 mA for 45 minutes. Thereafter, the gel was removed from the gel plate, washed with distilled water (3 repetitions of 20 minutes each), and soaked in the Gelcode Blue Reagent for approximately 1 hour for staining. Thereafter, the gel was washed with distilled water, the extent of discoloration was inspected, and the gel was then photographed. Fig. 7(A) shows the results of the experiment carried out using representative peptides.

Subsequently, purified proteins were identified via Western Blotting.

Electrophoresis was carried out using the fraction Ds obtained in Example 2 in the manner described above, except that the amount of the sample applied to 4% to 20% polyacrylamide gradient gel (Mini-PROTEAN TGX gels, BioRad) was changed to 10 µl Thereafter, the gel was removed from the gel plate and washed with a transfer buffer, which was prepared by mixing a solution of 36 g of Tris and 43.2 g of glycine in 2.4 liters of distilled water with 600 ml of methanol. After 4 sheets of filter paper, a membrane, gel, and 4 sheets of filter paper had been mounted in that order on the transfer device, the protein transfer was performed at 80 mA for 90 minutes. The filter paper was soaked in a transfer buffer and the membrane was soaked in methanol before use. After the transfer, the membrane was subjected to treatments described below: (1) soaking of the membrane in a 5% skim/PBS solution for 1 hour for impregnation; (2) impregnation thereof with a primary antibody (anti-human IgA α-chain goat antibody) solution for 1 hour; (3) washing thereof with PBST (3 repetitions of 10 minutes each); (4) impregnation thereof with a secondary antibody (HRP-labeled anti-goat IgG mouse antibody) solution for 1 hour; and (5) washing thereof with PBST (3 repetitions of 10 minutes each). The membrane thus treated was subjected to color development with the use of ChemiLumi One and then photographed. Fig. 7(B) shows the results of the experiment carried out with the use of representative peptides.

As shown in Fig. 7(A), the acidic elution fractions (fraction Ds) eluted as a result of chromatography in Example 2 were detected as smear bands spanning from 130 to 180 kDa, which were substantially similar to the IgA protein preparation as a result of protein staining, following SDS-PAGE. When Opt2 was used, a band was detected at a position around 25 kDa. When the peptide synthesized in Example 1 was used, however, no or substantially no such band was detected.

As shown in Fig. 7(B), in contrast, smear bands of the acidic elution fractions (fraction Ds) eluted as a result of chromatography in Example 2 and of the standard IgA preparation were observed at a position 300 kDa or higher, in addition to the 130- to 180-kDa bands described above, as a result of Western blotting using anti-human IgA antibodies. While IgA in the serum is mainly monomeric (serum IgA), a portion of IgA is dimeric (secretory IgA) linked by a J-chain. Thus, such two bands are detected. Also, these two bands were detected in the acidic elution fractions (fraction Ds) eluted as a result of chromatography in Example 2. This indicates that the column according to the present invention on which the IgA-binding peptide is immobilized can be used for purification of both the serum IgA and the secretory IgA.

The results demonstrate that peptides synthesized in Example 1 are specific for human IgA and highly useful as affinity ligands for purification. The peptides according to the present invention are useful not only as reagents for detection or purification of human IgA but also as standard purification systems for human IgA antibody drugs that are expected as novel antibody drugs in the future.

### Industrial Applicability

The present invention provides a peptide capable of specifically or selectively binding to human IgA. Such peptide is industrially useful for IgA purification when producing IgA as an antibody drug and for analysis of IgA.

### Free Text of Sequence Listing

### SEQ ID NOs: 1 to 13: IgA-binding peptides

All publications, patents, and patent applications cited herein are incorporated herein by reference in their entirety.

## Claims

1. A peptide, which comprises an amino acid sequence consisting of 14 to 18 amino acid residues represented by Formula (I) and is capable of binding to human IgA:
H-(X₁)-V-C-L-S-Y-R-(X₂)-(X₃)-G-(X₄)-P-(X₅)-C-(X₆)-(X₇)-(X₈) (I) (SEQ ID NO: 1)
wherein
H represents a histidine residue,
V represents a valine residue,
C represents a cysteine residue,
L represents a leucine residue,
S represents a serine residue,
Y represents a tyrosine residue,
R represents an arginine residue,
G represents a glycine residue,
P represents a proline residue, and
each X is independently selected from among the following:
X₁ represents a glutamine residue or a methionine residue;
X₂ and X₃ each independently represent an arbitrary amino acid residue other than cysteine or
either or both thereof are deleted;
X₄ represents an arginine residue or a glutamine residue;
X₅ represents a valine residue or a threonine residue;
X₆ represents a phenylalanine residue or a tyrosine residue;
X₇ represents a serine residue or is null; or
X₈ represents a leucine residue, a threonine residue, or is null,
provided that, when X₁ represents a methionine residue, X₇ represents a serine residue or is null, and X₈ is null.

2. The peptide according to claim 1, which comprises an amino acid sequence consisting of 16 to 18 amino acid residues represented by Formula (II) and is capable of binding to human IgA:
H-Q-V-C-L-S-Y-R-(X₂)-(X₃)-G-(X4)-P-(X₅)-C-(X₆)-S-(X₈) (II) (SEQ ID NO: 2)
wherein
H represents a histidine residue,
Q represents a glutamine residue,
V represents a valine residue,
C represents a cysteine residue,
L represents a leucine residue,
S represents a serine residue,
Y represents a tyrosine residue,
R represents an arginine residue,
G represents a glycine residue,
P represents a proline residue,
F represents a phenylalanine residue, and
each X is independently selected from among the following:
X₂ and X₃ each independently represent an arbitrary amino acid residue other than cysteine or
either or both thereof are deleted;
X₄ represents an arginine residue or a glutamine residue,
X₅ represents a valine residue or a threonine residue,
X₆ represents a phenylalanine residue or a tyrosine residue, or
X₈ represents a leucine residue or a threonine residue.

3. The peptide according to claim 1 consisting of either of the following amino acid sequences:
HMVCLSYRGRPVCF (SEQ NO: 3); or
HMVCLSYRGRPVCFS (SEQ ID NO: 4).

4. The peptide according to 1 or 2 consisting of any of the amino acid sequences 1) to 8):
1) HQVCLSYRGRPVCFSL (SEQ NO: 5);
2) HQVCLSYRGQPVCFSL (SEQ ID NO: 6);
3) HQVCLSYRGRPTCFSL (SEQ ID NO: 7);
4) HQVCLSYRGRPVCYSL (SEQ NO: 8);
5) HQVCLSYRGRPVCFST (SEQ ID NO: 9);
6) HQVCLSYRGQPVCFST (SEQ ID NO: 10);
7) HQVCLSYRGRPTCFST (SEQ ID NO: 11); or
8) HQVCLSYRGQPTCFST (SEQ ID NO: 12).

5. The peptide according to any one of claims 1 to 4, which forms a disulfide bond between two cysteine (C) residues.

6. The peptide according to any one of claims 1 to 5, which binds to serum (monomeric) IgA and secretory (dimeric) IgA.

7. The peptide according to any one of claims 1 to 6 comprising a label bound thereto.

8. A fusion protein consisting of the peptide according to any one of claims 1 to 7 and a protein which is linked to the peptide.

9. An immobilized peptide, which comprises the peptide according to any one of claims 1 to 7 bound to a solid phase.

10. A nucleic acid encoding the peptide according to any one of claims 1 to 7.

11. A method for purifying IgA comprising binding the peptide according to any one of claims 1 to 7 or the immobilized peptide according to claim 9 to IgA and releasing the bound IgA to collect the released IgA.

12. A method for detecting IgA comprising binding IgA in a sample to the peptide according to any one of claims 1 to 7 or the immobilized peptide according to claim 9 and detecting the bound IgA.

13. A kit for analyzing or purifying human IgA comprising at least one of the peptides according to any one of claims 1 to 7 or the immobilized peptide according to claim 9.

14. An IgA separation column comprising the immobilized peptide according to claim 9.
